# EUROPEAN PATENT APPLICATION

(11) **EP 2 230 233 A1**
(43) Date of publication of application: **22.09.2010**
(21) Application number: 07866968.6
(22) Date of filing: 27.12.2007
(51) Int. Cl.: C07D 235/08, A61K 31/4184, A61P 31/00

(54) **1,3-DIALKYL-BENZIMIDAZOLE HALOGENIDES EXHIBITING REGENERATION, ANTI-INFLAMMATORY AND ANTIMICROBIAL ACTIVITY**

(71) Applicant: Averin, Konstantin Mihailovich, Rostovskaya obl. 346900 (RU); Solodunov, Yuriy Yuryevich, Rostov-na-Donu 344048 (RU); Stradomskiy, Boris Vitalyevich, Rostov-na-Donu, 344052 (RU)
(72) Inventor: STRADOMSKIY, Boris Vitalyevich, Rostov-na-Donu 344052 (RU)
(74) Representative: Einsel, Martin
(86) International application number: PCT/RU2007/000744
(87) International publication number: WO 2009/084974

(57) **Abstract**

The invention relates to medicine, veterinary, and cosmetology, in particular, to medications used to accelerate tissue regeneration processes, particularly in the presence of inflammatory processes attended by infection with pathogenic microflora, and may be used in pharmaceutics and cosmetology.

The invention consists in 1,3-dialkylbenzimidazole halogenides exhibiting regenerative, anti-inflammatory, and antimicrobial activity of the general formula: 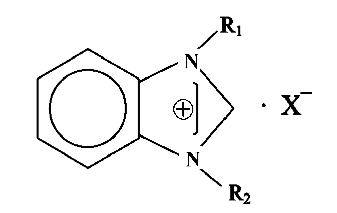 wherein: R₁ and R₂ = Alk,
X⁻ = F⁻, Cl⁻, Br⁻, J⁻.

## Description

### FIELD OF THE INVENTION

The invention relates to medicine, veterinary, and cosmetology, and particularly to medications used for accelerating regeneration of tissues, especially in the presence of inflammatory processes attended by infection with pathogenic microflora, and may be used in pharmaceutics and cosmetology.

### BACKGROUND OF THE INVENTION

Panthenol (2,4-dioxy-N-(3-oxypropyl)-3,3-dimethylbutyramide) is known to be widely used in pharmaceutics and cosmetology. It is used as a medication activating regenerative processes, accelerating the healing of wounds, burns, trophic ulcers, and so on (M.D. Mashkovsky, "Medications," Vol. 2, 14th Edition, Moscow, 2003, p. 90).

This medication is disadvantageous because of its low efficiency against serious inflammations and also its lack of antimicrobial activity, which makes it little effective against inflammations complicated by microbial infection of the wound.

The closest chemical compounds similar in purpose to the claimed 1,3-dialkylbenzimidazole monohalogenides are 1,2,3-trialkylbenzimidazole triiodides displaying antimicrobial activity (SU 770,021, C 07 D 235/08, A 61 K31/415, published).

The prior art compounds are disadvantageous because they comprise a triiodide ion containing active iodine J₂. Administration of active iodine medications is likely to cause an allergic reaction or symptoms of iodism. Besides, active iodine medications cause skin coloring that sharply restrict their use in cosmetology. In addition to these disadvantages, the immediate prior art invention has a significantly smaller number of compounds in this class and no fluorides, chlorides, and bromides of 1,2,3-trialkylbenzimidazole.

### SUMMARY OF THE INVENTION

The object of the invention that defines its purpose is developing new medications having a pronounced pharmacological activity on the basis of a cation of 1,3-dialkylbenzimidazole in which monohalogenides (for example, a fluoride, chloride, bromide or iodide), rather than polyhalogenides (for example, triiodide, dibromiodide, and so on) are used as the anion.

The technical result of the claimed invention that can be achieved by performing the same is production of a group of monohalogenide compounds of 1,3-dialkylbenzimidazole that exhibit regenerative, anti-inflammatory, and antimicrobial activity.

The idea of the claimed invention, which is aimed at developing substances exhibiting regenerative, anti-inflammatory, and antimicrobial activity of the general formula: 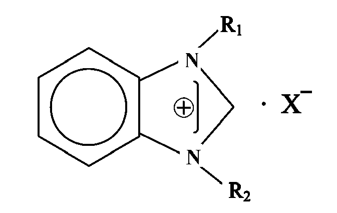 wherein: R₁ and R₂ = Alk, consists in that X = F⁻, Cl⁻, Br⁻, or J⁻.

### BRIEF DESCRIPTION OF THE DRAWING

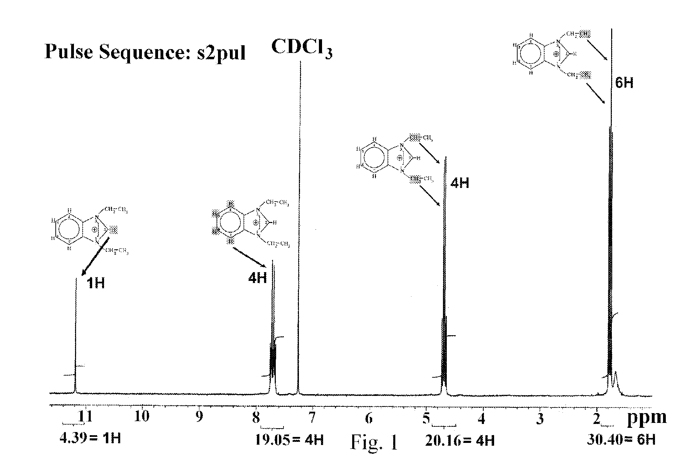

FIG.1 shows a **PMR (NMR-N') spectrum of 1,3-diethylbenzimidazole monoiodide in deuterochloroform.**

### DESCRIPTION OF AN EMBODIMENT OF THE INVENTION

The possibility of the invention being performed is supported by the following example:

### Example

1,3-diethylbenzimidazole iodide. 59 g (0.5 M) of benzimidazole and 68 g (0.5 M) of trihydrated sodium acetate are added to 150 ml of o-xylene, 156 g (1 M, 79.6 ml) of ethyl iodide are added to the mixture, and the reaction mixture is then boiled for 3 hours. The mixture is then cooled on ice, the resultant crystalline product is washed with ice water, filtered off, and dried for 24 hours at +55°C. Small crystals of white to light-yellow or light-pink color with a melting point of 242° to 244°C are produced at a yield of 74%. The structure of the compound obtained is confirmed by elementary analysis and the PMR spectrum (see: FIG. 1). N 9.23 % and J 43.7 % was found, and N 9.27 % and J 42.0% calculated.

Microorganism strains occupying different systematic positions, in particular, gram-positive Stafilococcus aureus cocci; gram-negative Escherichia coli bacilli; spore-producing Clostridium dificille bacilli, and then Pseudomonas aeruginosa and Salmonella typhi, were used for studying the antimicrobial activity of 1,3-diethylbenzimidazole iodide, a representative of the group of claimed compounds. Antimicrobial activity was studied according to the Official Pharmacopoeia (Official Pharmacopoeia of the U.S.S.R., 11th edition, Issue 2, Meditsina, Moscow, 1989).

The results obtained are given in Table 1. They show that the minimum suppressing concentration of 1,3-diethylbenzimidazole iodide for all the microorganism strains tested is equal to between 0.1 and 10.0 microgram/ml, that is, comparable to the effect of existing antibiotics.

The inflammation process was modeled by subplantar injections of carragheenin to rats. Twelve hours after carragheenin injections, 1,3-diethylbenzimidazole iodide was applied to the injection area in the form of a 1.5% ointment based on polyethylene glycol.

The results of studies of the anti-exudative and antipyretic effects of 1,3-diethylbenzimidazole iodide (Tables 2 and 3) show that the medication studied displays a distinct anti-inflammatory activity. This is also confirmed by the normalization dynamics of the biochemical characteristics of the inflammation process (Table 4).

The regenerative activity of the claimed compounds was studied on a model of a burn caused by light radiation on a specialized apparatus. Data yielded by studies of 1,3-diethylbenzimidazole iodide applied to a light burn are given in Table 5. They show that application of this compound stimulates a faster and more complete epithelization of the burn surface. Unless this medication is applied to a burn, a keloidal scar is produced on the burn area and no skin appendages are formed. The regenerative activity of 1,3-diethylbenzimidazole iodide is comparable with the effect of panthenol.

The following conditions are to be met for using the invention:

The substance of the claimed structure may be produced by a chemical method. The substance of the claimed structure may be used as an active principle exhibiting a regenerative, anti-inflammatory, and antimicrobial activity in medicine, veterinary, and cosmetology.

**Table 1. Identification of the antimicrobial effect of 1,3-diethylbenzimidazole iodide**

| **Medication concentration (microgram /ml)** | **Clostridium dificille** | **Escherichia coli** | **Pseudo-monas aeruginosa** | **Staphylococcus aureus** | **Salmonella typhi** |
|---|---|---|---|---|---|
| 10.0 | - | - | - | - | - |
| 1.0 | ± | - | ± | - | - |
| 0.1 | + | - | + | - | ± |
| 0.01 | + | + | + | + | + |

| | | | | | |
|---|---|---|---|---|---|
| + growth of the test microorganism; - no growth of the test microorganism; ± little or retarded growth. | | | | | |

**Table 2. Volume of an inflamed leg of a rat (cm³ × 10⁻¹; p = 10) in dynamics after inflammation modeling and application of 1,3-diethylbenzimidazole iodide**

| **Experimental group** | | **Observation time, hours** | | | |
|---|---|---|---|---|---|
| | | **Prior to inflammation, initial level** | **1** | **3** | **12** |
| Intact animals | cm³ × 10⁻¹ | 23±1 | 22±1 | 22±1 | 23±2 |
| | Decrease , % | - | - | - | - |
| Carragheenin | cm³ × 10⁻¹ | 23±1 | 35±2 | 35±2 | 30±2 |
| without treatment | Decrease, % | - | - | 0 | 14 |
| Carragheenin + 1,3-diethylbenzimidazole iodide | cm³ × 10⁻¹ | 23±1 | 36±2 | 30±1 | 25±2 |
| | Decrease, % | - | - | 17 | 31 |

**Table 3. Temperature difference (Δt°C) between an inflamed and intact legs of rats (p = 10) against the background of inflammation modeling and application of 1,3-diethylbenzimidazole iodide**

| **Experimental group** | | **Observation time, hours** | | | |
|---|---|---|---|---|---|
| | | **Prior to inflammation, initial level** | **1** | **3** | **12** |
| Intact animals | Δt °C | 1±1 | 2±1 | 1±1 | 1±1 |
| | Decrease, % | - | - | - | - |
| Carragheenin without treatment | Δt °C | 1±1 | 9±2 | 8±2 | 6±1 |
| | Decrease, % | - | - | 11 | 33 |
| Carragheenin + 1,3-diethylbenzodiazole iodide | Δt °C | 2±1 | 9±2 | 7±1 | 3±1 |
| | Decrease, % | - | - | 22 | 78 |

**Table 4. Inflammation process activity indicators in response to application of 1,3-diethylbenzimidazole iodide**

| **Experimental group** | **Observation hour** | **Support on the leg, redness,** | **ESR, mm/h r** | **CRB, mm recipitation** | **Sialic acids, Mmol/l** | **Fibrinogen, g/l** | **Leukocyte content, 10³/mm³** |
|---|---|---|---|---|---|---|---|
| Group 1. Intact animals | 1 | + | 4±1 | 0 | 1.7± 0.3 | 2.0± 0.3 | 6.5± 0.2 |
| Group 2. Carragheenin without treatment | 1 | - | 33±6 | >4 | 8.2± 0.9 | 13.5± 1.5 | 15.5± 1.2 |
| | 3 | - | 37±5 | >4 | 8.5± 1.7 | 12.6± 1.3 | 16.0± 1.2 |
| | 12 | - | 25±5 | >4 | 7.3± 0.9 | 10.2± 1.3 | 14.6± 0.8 |
| Group 3. Carragheenin + 1,3-diethylbenzimid -azole iodide | 1 | - | 25±5 | 3±1 | 6.5± 0.8 | 10.2± 1.2 | 12.5± 0.5* |
| | 3 | ± | 12±2* | 2±1* | 5.2± 0.6* | 5.1± 0.3* | 8.9± 0.2* |
| | 12 | + | 8±2* | 1±1* | 3.6± 0.4* | 3.1± 0.5* | 7.5± 0.5* |

| | | | | | | | |
|---|---|---|---|---|---|---|---|
| * established differences from Group 2 (p<0.05) | | | | | | | |

**Table 5. Percentage of fully epithelized burn wounds in response to application of 1,3-diethylbenzimidazole iodide**

| **Experimental group** | **25th day of** **observations** | **30th day of** **observations** |
|---|---|---|
| Burn without treatment, dry sterile bandage | 18 % | 25 % |
| Panthenol | 55 % | 85 % |
| 1,3-diethylbenzimidazole iodide | 57 % | 87 % |

## Claims

1. 3-dialkylbenzimidazole halogenides exhibiting regenerative, anti-inflammatory, and antimicrobial activity of the general formula: 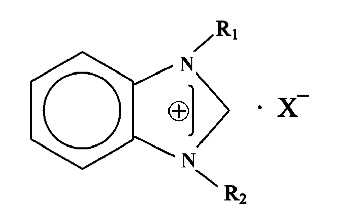 wherein: R₁ and R₂ = Alk,
X⁻ = F⁻, Cl⁻, Br⁻, J⁻.
